# EUROPEAN PATENT APPLICATION

(11) **EP 2 450 449 A1**
(43) Date of publication of application: **09.05.2012**
(21) Application number: 10190527.1
(22) Date of filing: 09.11.2010
(51) Int. Cl.: C12P 7/06, C12P 3/00

(54) **Process and apparatus for the production of alcohols**

(71) Applicant: Ineos Commercial Services UK Limited, Hampshire SO43 7FG (GB)
(72) Inventor: The designation of the inventor has not yet been filed
(74) Representative: Rickard, Timothy Mark Adrian

(57) **Abstract**

A process for the production of C2+ alcohols from a methane-containing feedstock which process comprises
a) optionally reacting in a pre-reformer the methane-containing feedstock in the presence of steam, where the steam plus CO₂ to methane molar ratio is less than 5:1;
b) reforming at least a portion of the methane-containing feedstock in a first reformer optionally in the presence of steam, where the steam plus CO₂ to methane molar ratio is less than 5:1 to produce a first product stream comprising CO, H₂ and CO₂;
c) optionally subjecting at least a portion of the first product stream and/or a portion of the methane containing feedstock to a reforming process in a second reformer in the presence of steam and oxygen to produce a second product stream comprising CO, H₂ and CO₂
d) subjecting the product streams to a bacterial fermentation process in a fermenter to produce a third product stream comprising an aqueous solution of at least one C2+ alcohol, nutrients and reaction intermediates and a fourth product stream comprising CO, H₂ and CO₂ at least 60% of the CO being converted;
e) recycling at least a portion of the fourth product stream to the methane-containing feedstock;
f) recovering at least a part of the at least one C2+ alcohols from the third product stream to leave a fifth product stream; and
g) recycling at least a part of the fifth product stream to the fermenter.

## Description

The present invention relates to a process and apparatus for the production of one or more C2+ alcohols. In particular, the present invention relates to a process for the production of one or more C2+ alcohols from a methane-containing feedstock via formation of carbon monoxide and hydrogen and subsequent fermentation of the carbon monoxide and hydrogen to one or more C2+ alcohols and recovery of said alcohols.

The production of alcohols from carbon oxides and hydrogen is well-known in the art. For example, a number of processes are known which use catalysts which are known to catalyse the reaction, including those based on Group VI metals, especially molybdenum, as described, for example in US 4,752,623 and US 4,831,060, and those based on mixed metal oxides, especially based on copper and cobalt containing catalysts, as described, for example, in US 4,122,110 and US 4,780,481. More recent publications include WO 2007/003909 A1, which also describes a process for the conversion of carbon oxide(s) and hydrogen containing feedstocks into alcohols in the presence of a particulate catalyst.

The catalytic routes generally produce a mixed alcohols product slate, including methanol, ethanol and heavier alcohols, especially propanol and butanols. The selectivity to the various alcohol products depends on the particular catalyst and process conditions employed and, although both methanol and the higher alcohols (ethanol and above) are usually formed in any particular reaction, the art generally seeks to maximise either methanol or the higher alcohols at the expense of the other.

There are also known processes for the conversion of carbon monoxide and hydrogen into C2+ alcohols based on fermentation processes using bacteria. Examples of fermentation processes can be found, for example, in WO 02/08438 and WO 00/68407, and are also described in DOE reports under DOE Contract Number DE-AC22-92PC92118, such as "Bench-scale Demonstration of Biological Production of Ethanol from Coal Synthesis Gas", Topical Report 5, November 1995.

In general such processes are much more selective for specific alcohols, such as ethanol, compared to catalytic processes, with much lower quantities, if any, of other alcohols being formed.

The carbon monoxide and hydrogen for such processes can be obtained by reforming of methane-containing feedstocks, such as natural gas, to produce a mixture of carbon monoxide, hydrogen and carbon dioxide (synthesis gas). A number of methane reforming processes and variants thereon are known in the art, as described in Hydrocarbon Processing April 2010, pages 33-42, by Bonneau, the principal types being:
(1) steam methane reforming (SMR), in which the methane containing feedstock is reformed in an externally fired reformer in the presence of >2:1 molar steam : methane ratio (usually > 2.5:1),
(2) autothermal reforming (ATR), in which the methane containing feedstock is reformed in the presence of steam and oxygen, and
(3) partial oxidation (POX), in which the methane containing feedstock is reformed in the presence of oxygen and relatively low or zero concentrations of steam

Significant variations on the above 3 processes are also known, and thus, for example, carbon dioxide can be added to steam methane reforming or autothermal reforming to adjust the ratio of hydrogen to carbon monoxide obtained. In a particular example, dry gas reforming is a variation of steam methane reforming in which the methane containing feedstock is reformed in the presence of significant concentrations of carbon dioxide and low or zero concentration of feed steam - the feed CO₂ has the effect of reducing the H₂:CO ratio and the low water content allows more effective conversion of CO₂ to CO.

In general, however, the ratio of hydrogen to carbon monoxide obtained is decreased in the order (1) > (2) > (3), with a typical SMR reformer (1) having an H₂:CO molar ratio of approximately 4.5:1 versus 2:1 for an ATR reformer (2) and 1.7 or 1.8:1 for a POX reformer (3). (Unless stated otherwise, all ratios herein are molar ratios)

Each of the above processes also produces carbon dioxide. As well as the highest carbon monoxide to hydrogen ratios, ATR and POX also result in the lowest carbon dioxide and methane in the resulting synthesis gas. Typically an SMR produces syngas with a molar ratio of CO₂:CO in the region of 0.35:1 versus 0.2:1 for an ATR and < 0.1:1 for a POX.

The concept of combined reforming has been described as to utilise the thermal energy of the ATR exit gas as a heat source for a steam-reforming reaction in a Gas Heated Reformer (GHR) instead of a conventional fired-reformer furnace of which two such arrangements, in series or in parallel, are described by Bonneau.

A further variant on the above Combined Reforming concept is that of the Lurgi Combined Reforming process, wherein an ATR is operated in combination and sequentially with an SMR to generate syngas suitable for use in petrochemical process such as large scale methanol production. The natural gas and any recycle gases plus steam are fed, in their entirety or partially, through an SMR operating at a low temperature, achieving partial conversion and then mixed with any bypassed reactants before passing into the ATR which is operated at a higher temperature ensuring high final feedstock conversion. In the case of methanol production often less than 50% of the natural gas feed is passed through the SMR. Other commercial variants exist where all the feed and recycle gases are fed in their entirety to the SMR before passing to the ATR or that the SMR and ATR are configured in parallel. Utilising different configurations and feed gas partitioning results in differing syngas product compositions often tailored for a downstream chemical application, such as methanol or Fischer Tropsch liquids manufacture.

In the combined reforming scheme where the ATR is combined with a Gas Heated Reformer either in a parallel or series arrangement, it is stated that this is a more energy efficient scheme than a combination with an externally fired SMR. A particular stated advantage of such a scheme is that oxygen loads are reduced and avoids requirement to remove large amounts of heat in the form of HP steam raising from the ATR effluent, however H₂:CO ratio is increased necessitating higher rates of CO₂ recycle to achieve the desired ratio for product use. In addition, such schemes are noted for issues with metal dusting corrosion, particularly at low steam to carbon ratios.

Thus, we see that variants of combined reforming schemes exist, each with particular features and advantages, and in context of this invention, we define Combined Reforming to include i) combination of an SMR and ATR in series where all the feed gas passes through the SMR before passing through the ATR unit, this arrangement being of particular suitability for use in the production of alcohols in a carbon efficient overall process and ii) the use of an ATR and GHR combination. The most favoured method of use in this invention would be the combination of SMR and ATR, in a series arrangement.

In theory, both catalytic and fermentation routes to higher alcohols (ethanol and heavier alcohols) may utilise CO₂ as a reactant for the production of the higher alcohols. However, in practise, both catalytic and fermentation routes to higher alcohols tend to be net producers of carbon dioxide.

In the case of catalytic conversions, such reactions may utilise the carbon dioxide via "direct" conversion or via co-occurrence of the water-gas shift reaction, CO₂ + H₂ ↔ CO + H₂O. However, whilst for methanol production, the production can occur directly from CO₂, most higher alcohol catalysts appear only to be able to react CO₂ via the shift reaction, and at the typical higher alcohol catalyst operating conditions of 250 - 400°C, the shift equilibrium favours CO₂ over CO - and results in the net production of CO₂ over the catalyst.

In the case of fermentation routes, the bacteria used for fermentation can produce alcohols according to either of the following 2 reactions

6CO + 3H₂O → C₂H₅OH + 4CO₂

2CO₂ + 6H₂ → C₂H₅OH + 3H₂O

However, the CO conversion is typically 70-90% per pass while the H₂ conversion is typically less than the CO conversion - therefore the fermentation is also a net producer of CO₂.

EP 2,017,346 is an example of a reformer scheme where fermentation is used to produce alcohols from synthesis gas. This document describes the advantages of dry gas reforming as a variant of SMR over alternative reforming technologies such as ATR & POX. One advantage was the lower rate of carbon dioxide emissions per unit of ethanol production for the SMR scheme over ATR & POX. It could therefore be inferred that if SMR & ATR were utilised in a combined reforming scheme that this advantage would be lost.

However, it has now been unexpectedly found that, contrary to this expectation, the integrated process for the production of C2+ alcohols from a methane-containing feedstock via intermediate formation of synthesis gas and subsequent fermentation operates most effectively as a Combined Reforming scheme.

Thus, in a first embodiment, the present invention provides a process for the production of C2+ alcohols from a methane-containing feedstock which process comprises
a) optionally reacting in a pre-reformer the methane-containing feedstock if it contains significant levels of C2 plus alkanes and other reforming catalysts fouling components such as recycled oxygenate species for example alcohols and organic acids in the presence of steam, where the steam plus CO₂ to methane molar ratio is less than 5:1;
b) reforming at least a portion of the methane-containing feedstock in a first reformer optionally in the presence of steam, where the steam plus CO₂ to methane molar ratio is less than 5:1 to produce a first product stream comprising CO, H₂ and CO₂;
c) optionally subjecting at least a portion of the first product stream and/or a portion of the methane containing feedstock to a reforming process in a second reformer in the presence of steam and oxygen to produce a second product stream comprising CO, H₂ and CO₂;
d) subjecting the product streams to a bacterial fermentation process in a fermenter to produce a third product stream comprising an aqueous solution of at least one C2+ alcohol, nutrients and reaction intermediates and a fourth product stream comprising CO, H₂ and CO₂ preferably at least 60% of the CO being converted;
e) recycling at least a portion of the fourth product stream to the methane-containing feedstock;
f) recovering at least a part of the at least one C2+ alcohols from the third product stream to leave a fifth product stream;
g) cooling at least a part of the fifth product stream; and
h) recycling at least a part of the cooled fifth product stream to the fermenter. According to the invention there is further provided apparatus for the production of C2+ alcohols from a methane-containing feedstock which apparatus comprises

a) optionally, a pre-reformer for converting any C2 plus alkanes present in the methane-containing feedstock and any recycled oxygenate species in the presence of steam, where the steam plus CO₂ to methane molar ratio is less than 5:1;
b) a first reformer for reforming at least a portion of the methane-containing feedstock optionally in the presence of steam, where the steam plus CO₂ to methane molar ratio is less than 5:1 to produce a first product stream comprising CO, H₂ and CO₂;
c) a second reformer for subjecting at least a portion of the first product stream and/or a portion of the methane containing feedstock to a reforming process in the presence of steam and oxygen to produce a second product stream comprising CO, H₂ and CO₂
d) a fermenter for subjecting the product streams to a bacterial fermentation process to produce a third product stream comprising an aqueous solution of at least one C2+ alcohol, nutrients and reaction intermediates and a fourth product stream comprising CO, H₂ and CO₂ preferably at least 60% of the CO being converted;
e) means for recycling at least a portion of the fourth product stream to the methane-containing feedstock;
f) means for recovering at least a part of the at least one C2+ alcohols from the third product stream to leave a fifth product stream;
g) means for cooling at least a part of the fifth product stream; and
h) means for recycling at least a part of the cooled fifth product stream to the fermenter.

The present invention thus provides a process for the production of C2+ alcohols. "C2+ alcohols" as defined herein, means ethanol and heavier alcohols, especially C2 to C6 alcohols, and most preferably C2 to C4 alcohols i.e. ethanol, propanol and butanols (isobutanol and n-butanol). C2+ alcohols can also be generally referred to as "higher alcohols".

In the process of the present invention, carbon dioxide and hydrogen in the product stream from the fermentation process are utilised as at least a portion of the feed to the reforming process. The reforming process is either in the substantial absence of steam, in which case it may be considered as dry-gas reforming, or a limited amount of steam is utilised, but with the proviso that where steam is also present in the feed to the reforming process the steam and CO₂ are present in a molar ratio of less than 5:1 (unless otherwise stated, as used herein all quantities and ratios are in moles).

The integrated process of EP 2,017,346 A1 utilised a pure methane feedstock, operated with a hydrogen excess and efficiently converted the carbon dioxide in the feed to the reforming process to carbon monoxide, resulting in a lower process inventory of carbon dioxide and smaller recycles with their associated energy use with the net result that less carbon dioxide ends up needing to be purged from the system giving lower net carbon dioxide production and high feedstock selectivity to the desired C2+ alcohol product. In contrast, the present invention, extensively modelled for a typical natural gas feedstock, efficient heat integration and achievable efficiencies from reformer flue gases, updates the carbon footprint data for a combined reforming scheme as well as providing updated comparable data for an SMR only scheme. The outcome being that despite having a lower feedstock selectivity to the desired C2+ alcohol product and having a higher carbon dioxide concentration entering the fermenter there is still a net reduction in the overall net carbon dioxide production through 3 mechanisms:
1. The excess hydrogen is reacted in each unit of the combined reformer with the CO₂ to form CO and water (the CO can be fermented to ethanol rather than CO₂ emitted to atmosphere)
2. The lower inventory of CH₄ in the recycle stream results in less energy use in the recycle stream
3. The reduction in steam use in the combined reformer reduces the combined reformer heating duty by means of reduced fuel gas use or reduced rate of oxygen addition.

In particular, it is possible to recycle all the CO₂ from the fermentation reaction to the reformer and still operate the system in hydrogen excess. The present invention also takes advantage of the fact that bacterial fermentation of synthesis gas to alcohols can be operated with relatively high carbon monoxide conversion, such that the product stream has a relatively low amount of carbon monoxide in it. This means that carbon monoxide can be economically recycled to the earlier reforming process. In a most preferred embodiment, this avoids the need for any specific separation of carbon monoxide from carbon dioxide in the product stream prior to recycle. Preferably, the fermentation step is operated to provide a conversion of CO of at least 70%, more preferably at least 80%.

In the integrated process of this invention the combined reformer is preferably operated at a pressure sufficient to feed the fermenter, in a scheme where no syngas compression is utilised, to overcome pressure losses due to reformer catalysts, heat recovery and syngas cooling steps and gas injection apparatus as required in the fermenter to ensure adequate gas dispersion into the liquid. Alternatively a syngas compression step could be utilised between the reformer and fermentation steps allowing flexibility in both reformer and fermenter operating pressures if so desired. It is however preferable that the reformer operates at a suitably higher pressure than the fermenter as this eliminates the requirement for an expensive compressor and the energy required for its operation.

The carbon monoxide conversion in a bacterial fermentation process is the result of a combination of a number of factors that can be controlled by the operator of the process. In general, the key requirements to obtain high CO conversion (>60%) are to ensure healthy bacteria and suitable contacting of the bacteria with the reactants. For a particular bacterial strain, this effectively means to ensure sufficient nutrients for the bacteria are provided, to ensure that the fermentation takes place in the correct temperature range, and to ensure sufficient gas contact with the bacteria, which is a function of gas pressure in the fermentation reaction, residence time in the fermentation reaction and reaction agitation. An additional parameter is the control of inert gases (by which is meant gas species which are not reactive compounds in the alcohol forming reactions) present in the feed gas to the fermenter. An example of an inert gas is nitrogen which is often found as a constituent of methane containing gases suitable as feedstock for this process. Inert gases would not typically be removed by a hydrogen selective membrane or particularly a Pressure Swing Adsorption (PSA) system for separation of hydrogen from the gaseous fourth product stream and will not be substantially converted in the combined reformer either, resulting in a continual increase in concentration. This concentration increase has several effects, 1) it increases the amount of inert gas being recycled thus adversely impacting on energy requirements for any recycle gas compression or heat duty for the reforming step, 2) increased concentration of inert gas reduces the partial pressure of the reacting gases , H₂, CO and CO₂ in the fermenter affecting mass transfer rates of the gas into the liquid phase and thence to the bacteria and 3) as a non reacting species it increases the volume of gas passing through and requiring to be disengaged from the fermenter liquid resulting in either higher levels of liquid entrainment or a requirement to have an increased diameter of fennenter.

In a most preferred embodiment, inert gas levels in the fermenter feed are controlled at an acceptable level to minimise effects as described above by means of a purge stream taken from the fourth gaseous product stream preferably prior to any hydrogen separation step as this reduces the vapour volume to be subject to hydrogen separation step. This purge stream containing purged inert gases will also contain H₂, CO, CO₂ plus other trace gases. This stream is preferably utilised as a fuel gas for the process. Depending on the level of inert gas entering the process with the methane containing feedstock and the controlling inert gas concentration being used, the amounts of CO and CO₂ being purged can amount to a significant portion of the overall carbon dioxide emission for the whole process. In a preferred embodiment where there is a requirement to reduce the alcohol process carbon footprint there are two methods by which this can be achieved namely i) by reducing the level of nitrogen (the most common inert gas) present in the methane containing feedstock to the process by means of utilising known membrane systems such as that for nitrogen separation from natural gas as available from Membrane Technology & Research Inc (Publication reference; "Nitrogen-rejecting membranes to increase gas heating value and recover pipeline natural gas : A simple wellhead approach", A Jariwala; K. Lokhandwala of MTR Inc USA) and ii) treating the purge stream in a similar manner with a nitrogen separation membrane of either glassy or rubbery type (Publication reference; Membrane Systems for Nitrogen Rejection by K Lokhandwala et al of MTR Inc USA) that will allow a reduced nitrogen content stream containing H₂, CO & CO₂ to be recovered for recycle to the reformer(s).

For a particular reaction and desired production rate such factors may be optimised by the person skilled in the art. In the event that conversion falls below 60% (or a higher threshold if required), then conversion can be increased again by acting on one of these parameters as might be necessary, for example, by increasing agitation rate, thereby increasing gas contacting with the bacteria.

Typically, the selectivity (based on total CO converted and on a non CO₂ basis) to higher alcohols of the fermentation process is at least 60%, especially at least 75%, and most preferably at least 90%. (CO₂ is a net reaction product in the conversion of CO to ethanol e.g. 6CO + 3H₂O → C₂H₅OH + 4CO₂. Selectivity on a non CO₂ basis relates to the conversion of CO to ethanol compared to methanol or alkanes)

Suitably, at least 60% of the carbon monoxide and 60% of the carbon dioxide in the gaseous fourth product stream are recycled, more preferably at least 80% of the carbon monoxide and 80% of the carbon dioxide in the gaseous fourth product stream are recycled and most preferably at least 85% of the carbon monoxide and 85% of the carbon dioxide in the gaseous fourth product stream are recycled to the reforming process of step (a). Whilst there are numerous "claims" in the literature for "high conversion" catalytic processes for the production of alcohols from synthesis gas, it is not believed that such processes can be operated at high conversion and high selectivity to higher alcohols. In particular, as conversion is increased, the selectivity of the catalyst systems to alcohols compared to alkanes is diminished.

US 4,831,060, for example, exemplifies only CO conversions of less than 40%. However, without high CO conversion the amount of carbon monoxide remaining in the gaseous fourth product stream of the present invention would be relatively high, and it would be necessary to separate at least some of the carbon monoxide from the carbon dioxide prior to recycle to maintain a driving force for CO₂ conversion to CO in the reforming process. This is described, for example, in SRI Report "Dow/Union Carbide Process for Mixed Alcohols from Syngas", PEP Review Number 85-1-4, in which carbon monoxide is separated from the recycle stream and recycled to the catalytic alcohols production process.

The preferred reforming processes according to the present invention are dry gas reforming and combined reforming (with steam limited as defined). A particularly preferred process is sulphur passivated reforming. Sulphur passivated reforming (SPARG) is described in Hydrocarbon Processing, January 1986, p. 71-74 or Oil & Gas Journal, Mar 9, 1992, p. 62-67. In such a process, sulphur is added to passivate the reforming catalyst. The sulphur reduces coke formation, which can otherwise be a problem. It is reported that the sulphur blocks the large sites (which are required to form coke) but leave the small sites open which allow reforming to continue.

The SPARG process is not believed to have been widely utilised for formation of synthesis gas. Without wishing to be bound by theory, it is believed that this may be:
(1) because most processes which use synthesis gas require higher H₂:CO ratios than are obtained by SPARG reforming, and
(2) because sulphur is generally a catalytic poison, which means it needs to be removed prior to any subsequent processing of the synthesis gas formed. This is achieved by converting sulphur species like mercaptan via a hydrogenation reaction over a catalyst creating hydrogen sulphide which is subsequently easily captured by passing through an absorbent such as zinc oxide.

In contrast to catalytic systems, bacterial fermentation processes have been found to be tolerant to sulphur present in the feed

Not only is there no need to remove sulphur prior to the fermentation step, therefore, but the sulphur can be readily recycled in the fourth product stream. However, in practice it is recognised that there will be an optimum level of sulphur species to be maintained in the process and as such it may be necessary to control by the means described above the amount of sulphur entering with the feedstock prior to the combined reformer or being recycled in the gaseous fourth product stream.

Where steam is present, the preferred steam:CO₂ molar ratio is less than 2:1, most preferably less than1:1. Lower steam:CO₂ molar ratios in the case of combined reforming have been found to result in lower efficiency of CO₂ conversion during the reforming step, higher steady state CO₂ concentration in process, but results in overall less CO₂ emissions for the process per Tonne of ethanol product compared with an SMR under dry reforming conditions.

The combined reforming process also produces H₂O. Advantageously, this water may also be used, following pre-treatment for removal of or reduction of deleterious components to the fermentation reactions if required, as part of the fermentation medium in the subsequent fermentation step. In addition, this water may also be used as feed to a process steam generating unit, with this being the preferred option for the majority of the water recovered downstream of the reformer which includes the water generated from the reforming reactions. Thus, in the process of the present invention, all the products of the synthesis gas production may be utilised in an energy efficient manner with minimal requirements for waste water treatments and disposal.

The process according to the present invention operates with excess hydrogen. In one embodiment, it is preferred to separate at least some of the hydrogen in the gaseous fourth product stream. As well as providing a source of a fuel gas (which can be used to fire the SMR, for example, saving further energy costs) this results in a net reduction in recycle rates to the reformer. Any suitable separation technique may be utilised. A Pressure Swing Adsorption system, especially configured for hydrogen removal is most preferred as this results in less loss of carbon species such as CO and CO₂ to fuel gas and thence as a carbon dioxide emission through combustion than at least some alternatives. Any suitable methane-containing feedstock may be utilised.

The most preferred feedstock is natural gas (which may or may not also include inherent quantities of carbon dioxide, nitrogen, higher hydrocarbons and sulphur species), but other suitable feedstocks include landfill gas, bio digester gas and associated gas from crude oil production and processing.

As noted previously, the present invention also takes advantage of the fact that bacterial fermentation of synthesis gas to alcohols can be operated with relatively high carbon monoxide conversion, such that the product stream has a relatively low amount of carbon monoxide in it. Not only does this mean that carbon monoxide in the fourth product stream can be economically recycled to the earlier reforming process, but the lower carbon monoxide in the feed to the reforming process favours further the conversion of carbon dioxide according to the reforming equilibrium (CO₂ + CH₄ ↔ 2CO + 2H₂).

In a most preferred embodiment, the process of the present invention is operated at elevated pressure in both the reforming and fermentation steps. Preferably the pressure is in the range 2 to 20 barg for both steps. The pressure is preferably based on the optimum pressure for the fermentation step, and the reforming process operated at a suitably higher pressure to allow for inherent pressure loss between process steps as outlined previously, to provide the product stream at the required pressure for the fermentation step, and with minimal compression required for the recycle of the fourth gaseous product stream to the reforming process. One further advantage of SPARG technology, for example, is that it may be operated across a wide range of pressures dependent on the downstream processing required without significant variations in product distribution.

The fermentation process may use any suitable bacteria. The preferred fermentation process uses acetogenic anaerobic bacteria, especially a rod-shaped, gram positive, non-thermophilic anaerobe. Useful bacteria according to this invention include *Acetogenium kivui*, *Acetobacterium woodii, Acetoanaerobium noterae, Clostridium aceticum, Butyribacterium methylotrophicum, Clostridium acetobutylicum, Clostridium thermoaceticum, Eubacterium limosum, Peptostreptococcus productus, Clostridium ljungdahlii, Clostridium carboxydivorans, Clostridium ragsdalei and Clostridium autoethanogenum.* In addition, other acetogenic anaerobic bacteria may be selected for use in the process of the invention. It will also be appreciated that a mixed culture of two or more bacteria may be used in the process of the present invention. Suitable microorganisms and/or growth conditions may include those disclosed in US 2007/0275447 which discloses a biologically pure culture of the microorganism *Clostridium carboxidivorans* having all of the identifying characteristics of ATCC no. BAA-624; and US2008/0057554 which discloses a biologically pure culture of the microorganism *Clostridium ragsdalei* having all of the identifying characteristics of ATCC No. BAA-622. A further particularly suitable bacteria is *Clostridium ljungdahlii*, especially suitable strains or mixtures thereof , being *Clostridium ljungdahlii PETC*, *Clostridium ljungdahlii ERI2, Clostridium ljungdahlii C01, Clostridium ljungdahlii O-52. Clostridium ljungdahlii* and processes using such bacteria are described in DOE report "Bench-scale Demonstration of Biological Production of Ethanol from Coal Synthesis Gas", Topical Report 5, November 1995 (DOE Contract Number DE-AC22-92PC92118) and in patent applications WO 98/00558, WO 00/68407 and WO 02/08438. The fermentation process generally comprises contacting the product stream comprising CO, H₂ and CO₂ with the bacteria in the presence of a nutrient medium in a suitable reactor, for example a continuously stirred tank reactor (CSTR). Suitable temperatures and pressures are dependent on the bacteria and other process conditions used, but typical temperatures for the fermentation are between 25°C and 85°C, especially 35°C to 45°C and typical pressures are in the range atmospheric to 20 barg, preferably 2 to 17 barg.

US 7,285,402 provides information about how to operate fermenters of use in the invention.

"Nutrient medium" is used generally to describe conventional bacterial growth media which contain vitamins and minerals sufficient to permit growth of a selected subject bacteria. Suitable nutrients are well-known, for example as described in US 7,285,402, WO 08/00558, US 5,807,722, US 5,593,886 and US 5,821,111.

The agitation rate may be selected depending on the reaction vessel and robustness of the bacteria. In particular, the reaction mixture is generally agitated at a suitable rate to ensure adequate gas dispersion and substantial avoidance of agglomeration of dispersed gas bubbles whilst minimising damage to the bacterium cells caused by any moving parts e.g. stirrer tips.

In practice this usually means that for a larger unit agitated with a stirrer a smaller RPM (revolutions per minute) is used than for a corresponding smaller unit (for a fixed RPM, the tip speed of a larger agitator is faster than that of a smaller agitator). Speeds of 20 to 1000 RPM are typical, with larger units operating at the lower rates.

The residence time may also be selected depending on the particulars of the reaction, and in order to obtain the desired conversion. The residence time is usually in the range 5 seconds to 20 minutes, and most typically in the range 10 seconds to 5 minutes.

Generally, the fermentation step produces a gas phase product comprising CO, H₂ and CO₂ (which forms the fourth product stream according to the present invention) and a liquid reaction broth comprising a mixture of fermentation bacteria, nutrients, alcohols, and by-products, such as acetic acid, in >95% water. The liquid reaction broth is usually removed from the fermenter and filtered to remove cells and other solids, then distilled to produce a more concentrated alcohol/water product mixture and a fifth product stream comprising nutrients, water and acetic acid which is returned to the fermenter. C3 and higher alcohols co-produced during the fermentation recovered during the distillation process optionally may be recycled to the water saturation unit, vaporised and mixed with the methane containing feed gas to the reformer. Generally less than 3wt% of 4^{th} product stream may be co-produced as C3 or higher alcohols.

Distillation is a long established technique for the recovery of ethanol with several schemes developed in academia or used in industry such as "Energy saving distillation designs in ethanol production" by M. Collura & W Luyben published in Ind Eng Chem Res 1988 Vol 27 p1686-1696, which identifies several suitable designs including split feed twin tower concepts, also found in US 5,035,776 and US 4,306,942.

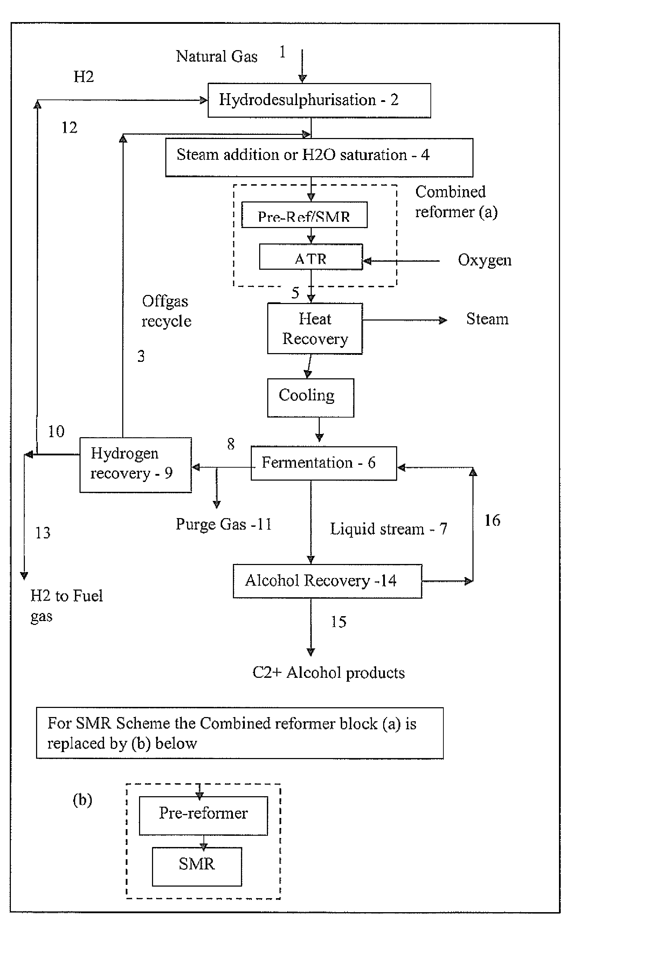

The invention will now be described with reference to Figure 1, which shows in schematic form a process for the production of alcohols from a methane-containing feedstock according to the process of the present invention.

In particular, Figure 1 shows a combined reforming process in which a methane containing feedstock (1) is first passed through a hydrodesulphurisation treating step (2) then mixed with a recycle stream (3) comprising carbon dioxide, carbon monoxide and hydrogen. Steam, if required, may be provided directly from a boiler or turbine passout or preferably from a saturator (4). Reforming of the methane-containing feedstock produces a product stream (5) comprising CO, H₂ and CO₂, which is passed, after heat recovery and cooling, to a bacterial fermentation step (6) where it is converted in the presence of a suitable bacteria to produce a third product stream (7) comprising one or more alcohols in the liquid phase and a gaseous fourth product stream (8) comprising CO, H₂ and CO₂, the fermentation step being operated to provide a conversion of CO of at least 60% (Separations steps in the fermenter not shown). The gaseous fourth product stream (8) comprising CO, H₂ and CO₂ is passed to a PSA (9) where a portion of the hydrogen contained therein is separated (10), to leave a stream comprising carbon dioxide, carbon monoxide and the remaining hydrogen which is recycled as stream (3). A controlled purge (11) removed upstream of the PSA from the fourth product stream is also taken to control the level of inerts such as nitrogen and argon in the feed to the fermenter and the recycle stream and is utilised as a fuel gas for the process. The separated hydrogen can be part utilised for the methane treatment step (12) or used as a fuel gas for the SMR refonner unit of the combined reformer (13). The liquid third product stream (7) is passed to an alcohol recovery step (14) wherein C2+ alcohols are recovered (15) and at least a portion of the fifth product stream (16) is recycled back to the fermenter (6).

The fifth product stream is cooled before it enters the fermenter. Typically this is done by passing it through a heat exchanger where it exchanges heat with the third product stream. Fifth product stream exiting the heat exchanger may have a temperature in the range of 45 to 50°C. In at least some embodiments of the invention the cooled fifth product stream is subject to a further cooling process for example in a trim cooler, which may be cooled by water, to a temperature of 40°C or lower.

### Examples

Integrated processes for reforming and fermentation according to Figure 1 have been modelled using Aspen:
- the combined reformer includes a pre-reformer modelled as a stoichiometric adiabatic reactor followed by a steam methane reformer and an autothermal reformer in series modelled as equilibrium reactors with the SMR specified with an outlet temperature of 770°C and the ATR specified with an outlet temperature of 1000°C
- The following ethanol forming reactions are modelled within the fermenter

   6CO + 3H₂O → C₂H₅OH + 4CO₂

   2CO₂ + 6H₂ → C₂H₅OH + 3H₂O

In the present Examples, a H₂ conversion of half that of the CO conversion is used, providing a net reaction of:

6CO + 1.5 H₂O + 3H₂ → 1.5 C₂H₅OH + 3CO₂

The overall CO conversion used is 90% and includes some allowance for generation of minor quantities of higher alcohols.

All modelling assumes the same ethanol production rate, a hydrogen recovery of 78% in the PSA and a control level of nitrogen as measured in the third product gaseous stream leaving the fermenter of 5 mole% and a natural gas feed composition as described in Table 1. A key feature of combined reforming schemes is the requirement to provide oxygen for the ATR, at scale. This is usually provided at high purity from cryogenic process such as those commercially available from Air Products or Linde or others. Electrical energy demand usually expressed as kWhr/te oxygen for such systems are largely dependent on scale and the vendor's particular technology. For the purpose of this invention a representative conservative value close to published data from Air Products (presentation entitled "ITM oxygen for gasification economic improvement: status "from 7th European Gasification Conference held in Barcelona during April 2006) has been used as the base case for the combined reforming scheme of the invention. Furthermore the importance of electrical demand when included in calculating carbon efficiency of the invention is dependent on the electrical energy demand values assumed for oxygen production; the lower the value the greater the flexibility to allow more feedstock to bypass the SMR. Advances such as those described in Chemical Engineering Progress, Jan 2009, Pages 6-10 and an Air Products news item published on their website as http://www.airproducts.com/PressRoom/CompanyNews/Archived/2009/21May2009b.htm indicate that significant reductions may be possible of around 30%. For illustrative purposes in the comparative examples a reduction of 25% in electrical demand is utilised in the data presented in Table 2 for - Combined Reforming -Case B.

**Table 1.**

| **Natural Gas Component** | **Mole %** |
|---|---|
| Methane | 94.9 |
| Ethane | 2.5 |
| Propane | 0.2 |
| n-Butane | 0.08 |
| Nitrogen | 1.6 |
| Oxygen | 0.01 |
| Carbon Dioxide | 0.7 |
| Mercaptan (methyl) | 0.01 |
| **Total** | **100** |

### Comparative Examples

In a first comparative example, a SMR operating under dry reforming conditions is compared against a combined reforming process scheme also operating under dry reforming conditions. A feed of natural gas, first subjected to hydrodesulphurisation, using hydrogen recovered from the hydrogen separation step, to convert the mercaptan to hydrogen sulphide which is then captured in a bed of ZnO then mixed with a recycle stream containing hydrogen, carbon monoxide, carbon dioxide plus residual levels of water and methane and trace alcohols before being passed via a water saturator operated to give the desired steam to methane ratio to either of the reforming blocks defined as (a) or (b) in Fig 1 under the feed conditions as specified in Table 2; Results Columns 1 & 2. In the SMR case a pre-reformer (modelled as an RSTOIC reactor is incorporated and operated at a feed temperature of 550°C in the presence of a catalyst to reform all organic compounds larger than methane). In the Combined Reformer case the pre-reformer is retained for comparative purposes, before the gas passes through an SMR operating at a low temperature <800°C before passing to an autothermal reformer where autothermal reforming occurs, through the addition of pre-heated oxygen. Those skilled in reforming will recognise that where the feedstock has few C2 plus alkanes then often a pre-reforming catalyst can be deployed as a first contact catalyst in a low temperature operating SMR. The reforming produces a product stream consisting of hydrogen carbon monoxide carbon dioxide water and a minor amount of methane in the vapour phase. The SMR is a fired reformer using predominately fuel gas generated from the process plus supplemental natural gas as per composition in Table 1. The flue gas after passing the catalyst reforming, tubes is still very hot, typically 950°C-1100°C, the range and efficient recovery of this heat is essential for an economic process. This heat is utilised for superheating steam, pre-heating reformer feed gases and pre-heating feed gas to the hydrodesulphurisation step plus pre-heating the combustion air and, if required, for raising further low pressure steam for process or electrical power generation use before the flue gas at typically 130°C to 160°C exits via a fan to the reformer stack. The initial product stream from the reformer is subjected to a heat recovery step which preferably generates high pressure saturated steam which is then superheated by the hot flue gases from the fired SMR before being passed though a high pressure steam turbine, with a portion of the steam, known as passout steam, from a turbine stage used to meet the thermal demand for the reboiler of the high pressure tower of the ethanol recovery distillation unit, the residual steam being further expanded with further passout of low pressure steam extracted for use in the process as a direct heating medium, the residue finally passing into a condensing turbine stage. The turbines generate electricity for use in the process and to provide electrical power for an air separation unit where required to do so.

After HP steam raising the reformer product stream is further cooled by heat interchange with recovered water in a water saturation step to generate the steam:methane ratio desired for the reformers. Other cooling steps are additionally incorporated such as the raising of additional low pressure steam, preheating of utility or reactant streams or use of process cooling water allowing the majority of the water to be subsequently separated to leave a second product stream at less than 40 °C comprising gaseous components and some residual water at the process conditions, typically <0.5 mole % (detailed separations not shown in Figure 1). A portion of the separated recovered water is used in the saturator with the balance being available to be passed (if desired) with the first product stream to the fermentation step where fermentation occurs at 1.1 MN/m² (abs) to produce 2 product streams:
- a gas stream (8) comprising hydrogen, carbon monoxide, carbon dioxide, inert gases such as nitrogen and a minor amount of methane, and
- a liquid stream (7) consisting of water ethanol and minor quantities of higher alcohols and other components as part of the biological reaction mixture such as nutrients and reaction intermediary species such as acetic acid.

The gas stream, depleted in CO and hydrogen but enriched by CO₂ during the fermentation, and also enriched in inert gases such as nitrogen due to its presence in the natural gas feed, is passed to a hydrogen separating step. A small purge of this gas is taken from this stream prior to the hydrogen separation step as a means of controlling inert gas levels in the fermenter. This purge stream in this example is used as a fuel gas for the process. Hydrogen is recovered using a PSA to deliver a very high purity gas allowing retention of the carbonaceous species to be recycled to the reformer. The amount of hydrogen abstracted can be optimised to suit the overall operation of the process and the recovery value of 78% utilised in this process configuration allows direct comparison of the different reformer schemes. This abstracted hydrogen is utilised as a fuel gas for the process and as a reactant for the hydrodesulphurisation step with addition sufficient to provide a small percentage excess over reaction requirements.

The liquid stream of crude alcohols is partly de-pressured during the membrane separation step within the fermenter unit operation (not shown in Fig 1) and further de-pressurised in a vessel allowing dissolved gases, H₂, CO and particularly CO₂ which is highly soluble to escape from the liquid before it is passed to the alcohol recovery step. Dissolved gases once released are captured in a process flare system resulting in a process emission of CO₂ after combustion. The alcohol separation step of this example was a distillation step implemented as two separate distillation towers operating under different pressures and liquid feed loadings as described previously for optimal energy use in the alcohol recovery system. A product ethanol drying step is also included within this recovery scheme generating essentially dry ethanol product suitable for use as motor transport fuel. Any higher alcohols present are also recovered separately from the distillation towers.

The modelling of the process also comprised recovery of all the fuel gas streams plus any supplemental requirements for fuel gas (supplied as natural gas of composition illustrated in Table 1) and their full combustion in the fired reformer furnace with heat recovery, (including but not limited to, reformer feeds preheating, air preheat and steam superheating), from the flue gas to give a stack exit temperature in the range 130-160°C, which is typical of such installations. Reformer stack emissions provide the dominant portion of the overall carbon dioxide emission but it is noted that where inert gas control is required a significant source of the stack emission plus flare emission is derived from the inert gas purge stream. In all the comparative examples provided, the plant is either electrically power balanced or in slight surplus such that an accurate assessment of the carbon emissions for the process could be determined including those for the operation of the air separation unit providing the oxygen for the ATR, which is a significant power user. The detailed comparison of results from modelling the above describes process in either an SMR configuration or as a combined reformer configuration are presented in Table 2 on basis of equal ethanol product production.

**Table 2**

| **Operating parameter** | **SMR** | **Combined Reformer** | **Combined Reformer Case A** | **Combined Reformer Case B** |
|---|---|---|---|---|
| Reformer exit temperatures (°C) | 920 | 770 & 1000 | 770 & 1000 | 770 & 1000 |
| Reformer Pressure (MN/m² (abs)) | 1.55 | 1.6 & 1.55 | 1.6 & 1.55 | 1.6 & 1.55 |
| ASU electical power use (kWhr/te O2) | 0 | 250 | 250 | 188 |
| H2O:CO2 reformer feed ratio -mole | 0.62 | 0.44 | 0.43 | 0.39 |
| % flow bypass around SMR | 0 | 0 | 5 | 17.5 |
| (Steam+CO2):CH4 ratio in reformer | 1.67 | 2.1 | 2.14 | 2.29 |
| 02 addition rate Kmol/te ethanol | 0 | 0.018 | 0.019 | 0.022 |
| % CO2 overall conversion across reformer(s) | 71.4 | 53.8 | 52.7 | 49.0 |
| CO2:CO ratio in syngas product | 0.16 | 0.33 | 0.35 | 0.41 |
| H2:CO ratio in syngas to fermenter | 1.24 | 0.94 | 0.92 | 0.86 |
| Te Nat Gas feed/Te Ethanol | 0.867 | 0.922 | 0.926 | 0.939 |
| Te CO2 emitted/te Ethanol | 0.850 | 0.807 | 0.815 | 0.819 |
| CO2 emitted in N2 control purge as wt % of total process emission. | 29.6 | 48.3 | 49.0 | 52.8 |
| CO recycled to reformer % molar | 90.7 | 89.0 | 89.0 | 89.4 |
| CO2 recycled to reformer % molar | 90.7 | 88.9 | 89.0 | 89.1 |

A second comparative example is provided for a combined reforming scheme to illustrate the effect of partial methane containing feedstock bypass of the SMR and the influence that electrical power requirements of the air separation unit has on the amount of bypass that can be utilised. Referring to Table 2 Results column - "Case A" represents a combined reformer scheme as broadly described in the first comparative example but with a portion of the feed bypassing the SMR. Referring to Results column - "Case B" an identical scheme but with a lower rate of electrical power demand for the air separation unit that provides the oxygen to the ATR is provided for comparative purposes.

A particular advantage of the process of the present invention is that the CO₂ emissions from the overall process are reduced, with an approximate 5% reduction observed in overall CO₂ emissions /te of ethanol for the combined reformer scheme (0.807 te/te) over the SMR scheme (0.850 te/te) despite the combined reformer scheme of this invention requiring a higher rate of feed gas per te of ethanol product. Table 2 shows that a significant portion of the gas feedstock can bypass the SMR of the invention without adversely impacting on the carbon efficiency of the combined reforming scheme in comparison to that of the SMR only design, however will be recognised that it may not be advantageous or optimal to have large proportions of gas feedstock bypassing the SMR as the rate of ethanol product generation per te of natural gas feed decreases and the carbon footprint does rise.

## Claims

1. A process for the production of C2+ alcohols from a methane-containing feedstock which process comprises
a) optionally reacting in a pre-reformer the methane-containing feedstock in the presence of steam, where the steam plus CO₂ to methane molar ratio is less than 5:1;
b) reforming at least a portion of the methane-containing feedstock and/or output from optional step a) in a first reformer optionally in the presence of steam, where the steam plus CO₂ to methane molar ratio is less than 5:1 to produce a first product stream comprising CO, H₂ and CO₂;
c) optionally subjecting at least a portion of the first product stream and/or a portion of the methane containing feedstock to a reforming process in a second reformer in the presence of steam and oxygen to produce a second product stream comprising CO, H₂ and CO₂;
d) subjecting the product streams to a bacterial fermentation process in a fermenter to produce a third product stream comprising an aqueous solution of at least one C2+ alcohol, nutrients and reaction intermediates and a fourth product stream comprising CO, H₂ and CO₂ preferably at least 60% of the CO being converted;
e) recycling at least a portion of the fourth product stream to the methane-containing feedstock;
f) recovering at least a part of the at least one C2+ alcohols from the third product stream to leave a fifth product stream;
g) cooling at least a part of the fifth product stream; and
h) recycling at least a part of the cooled fifth product stream to the fermenter.

2. A process as claimed in claim 1 where the bacterial fermentation process uses acetogenic anaerobic bacteria, especially rod-shaped, gram positive, non-thermophilic anaerobes.

3. A process according to claims 2 wherein the bacteria is at least one of *Acetogenium kivui*, *Acetobacterium woodii*, *Acetoanaerobium noterae*, *Clostridium aceticum*, *Butyribacterium methylotrophicum*, *Clostridium acetobutylicum*, *Clostridium thermoaceticum*, *Eubacterium limosum*, *Peptostreptococcus productus*, *Clostridium ljungdahlii, Clostridium carboxydivorans, Clostridium ragsdalei and Clostridium autoethianogenum*

4. A process as claimed in claim 2 wherein the bacteria comprises at least one of *Clostridium carboxidivorans* having all of the identifying characteristics of ATCC no. BAA-624 and *Clostridium ragsdalei* having all of the identifying characteristics of ATCC No. BAA-622.

5. A process as claimed in claim 1 or claim 2 wherein the bacteria comprises at least one of *Clostridium ljungdahlii*, especially *Clostridium ljungdahlii PETC*, *Clostridium ljungdahlii ERI2, Clostridium ljungdahlii C01*, *Clostridium ljungdahlii O-52.*

6. A process as claimed in any one of the preceding claims wherein step b) is a steam reforming process, the first reformer is a steam reformer and at least a part of the first product stream is subjected to step c).

7. A process as claimed in claim 5 wherein a portion of the H₂ of the fourth product stream is recovered and used as a fuel to fire the steam reformer.

8. A process as claimed in any one of the preceding claims further comprising extracting a purge stream comprising inert gas from the fourth product stream.

9. A process as claimed in claim 7 when dependent on claim 4 wherein the purge stream is recovered and used as a fuel to fire the steam reformer.

10. A process as claimed in any one of the preceding claims wherein at least a portion of the methane-containing feed stock is subjected to a hydrodesulphurisation treatment.

11. A process as claimed in claim 9 wherein a portion of the H₂ of the fourth product stream is recovered and utilised in the hydrodesulphurisation treatment.

12. A process as claimed in any one of the preceding claims wherein a portion of the water in the reformer product stream is recovered, cooled to below 50°C, optionally treated and utilised as a co-feed to the fermenter.

13. A process as claimed in any one of the preceding claims wherein at least a portion of the water in the reformer product stream is recovered, and utilised as a co-feed to a partial water vaporisation unit to saturate the natural gas feedstock to the required steam to methane ratio.

14. A process as claimed in any one of the preceding claims wherein the at least one C2+ alcohols are subjected to a distillation process.

15. A process as claimed in claim 12 wherein the distillation process comprises feeding a portion of the at least one C2+ alcohols to a first distillation column operating at a first pressure and to a second distillation column operating at a second pressure lower than the first pressure.

16. A process as claimed in claim 12 wherein at least a portion of material exiting overhead from the first distillation column is used as a heat source for the second distillation column.

17. A process as claimed in any one of the preceding claims wherein at least a part of the methane containing feedstock is subjected to sulphur passivated reforming.

18. A process as claimed in any one of the preceding claims wherein at least 80% of the carbon monoxide and 80% of the carbon dioxide in the fourth product stream are recycled.

19. A process as claimed in any one of the preceding claims wherein both the reforming and fermentation steps are operated at a pressure in the range 2 to 20 barg.

20. A process as claimed in claim 13 wherein C3 and higher alcohols recovered from the C2+ alcohols distillation step are at least partially recycled to the pre-reformer via the reformer feed gas water saturator.

21. A process as claimed in any one of the preceding claims wherein at least portion of the methane containing feedstock is subjected to a nitrogen content reduction step being subjected to reforming.

22. A process as claimed in claim 10 wherein at least a portion of the methane containing feedstock is subjected to a nitrogen content reduction step before being subjected to hydrodesulphurisation.

23. Apparatus for the production of C2+ alcohols from a methane-containing feedstock which apparatus comprises
a) optionally a pre-reformer for converting C2 plus alkanes present in the methane-containing feedstock and any recycled oxygenate species in the presence of steam, where the steam plus CO₂ to methane molar ratio is less than 5:1;
b) a first reformer for reforming at least a portion of the methane-containing feedstock and at least a portion of output from the optional pre-reformer optionally in the presence of steam, where the steam plus CO₂ to methane molar ratio is less than 5:1 to produce a first product stream comprising CO, H₂ and CO₂;
c) a second reformer for subjecting at least a portion of the first product stream and/or a portion of the methane containing feedstock to a reforming process in the presence of steam and oxygen to produce a second product stream comprising CO, H₂ and CO₂;
d) a fermenter for subjecting the product streams to a bacterial fermentation process to produce a third product stream comprising an aqueous solution of at least one C2+ alcohol, nutrients and reaction intermediates and a fourth product stream comprising CO, H₂ and CO₂ preferably at least 60% of the CO being converted;
e) means for recycling at least a portion of the fourth product stream to the methane-containing feedstock;
f) means for recovering at least a part of the at least one C2+ alcohols from the third product stream to leave a fifth product stream;
g) means for cooling at least a part of the fifth product stream; and
h) means for recycling at least a part of the cooled fifth product stream to the fermenter.
